(19) Europäisches Patentamt
European Patent Office
Office européen des brevets

(11) Publication number: **0 264 815 B1**

(12) **EUROPEAN PATENT SPECIFICATION**

(45) Date of publication of patent specification: **12.08.92**

(51) Int. Cl.5: **C12Q 1/00**, C12Q 1/28, C12Q 1/26, C12Q 1/32

(21) Application number: **87115036.3**

(22) Date of filing: **14.10.87**

(54) **Methods for selective measurement of amino acids.**

(30) Priority: **20.10.86 JP 247371/86**

(43) Date of publication of application:
**27.04.88 Bulletin 88/17**

(45) Publication of the grant of the patent:
**12.08.92 Bulletin 92/33**

(84) Designated Contracting States:
**AT BE CH DE ES FR GB GR IT LI LU NL SE**

(56) References cited:
**DE-A- 3 040 512**
**GB-A- 1 065 526**

**METHODS OF ENZYMATIC ANALYSIS, 3rd edition, 1985, pages 318-329,405-411, VCH Verlagsgesellschaft mbH, Weinheim, DE;**

(73) Proprietor: **ONO PHARMACEUTICAL CO., LTD.**
**No. 14, Doshomachi 2-chome Higashi-ku**
**Osaka-shi Osaka(JP)**

(72) Inventor: **Shimizu, Hiroshi**
**E-106, Aza-Miyasaka 108 Suisha-Shinden**
**Nada-ku**
**Kobe-shi Hyogo(JP)**
Inventor: **Sugiyama, Masayasu**
**26-46, Yamatedai 3-chome**
**Ibaraki-shi Osaka(JP)**
Inventor: **Taniguchi, Ken**
**F-108, Shin-ashiyakami 27-ban**
**Suita-shi Osaka(JP)**

(74) Representative: **Henkel, Feiler, Hänzel & Partner**
**Möhlstrasse 37**
**W-8000 München 80(DE)**

EP 0 264 815 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

### 1. Field of the Invention

The present invention relates to the selective measurement of specific amino acids. More particularly, it relates to methods for selective measurement of aromatic amino acids or branched-chain amino acids, which are characterized by coupling plural enzymes containing those specific for aromatic amino acids or containing those specific for branched-chain amino acids, respectively, to a sample, to produce hydrogen perioxide, and then measuring said hydrogen peroxide.

### 2. Prior Arts

Recently, it is understood that abnormal generation and use, of each amino acid occur in patients with liver diseases e.g. hypofunction of liver, thereby aromatic amino acids are greatly accumulated in blood, and therefore the ratio of aromatic amino acids to branched-chain amino acids in blood (The ratio is named "Fischer's ratio".) is highly changed in such patients as compared with normal condition. Furthermore, it is reported that by measuring Fischer's ratio, the degree of liver damage may be judged [cf. Fischer J. E. et al., Surgery, 80, 77 (1976)]. The therapy by amino acids-infusion has been recently carried out in hepatic coma induced by abnormality of amino acids.

It is essential to measure Fischer's ratio in judgement of the degree of liver damage and in monitor of amino acid in the therapy by amino acids-infusion, and its significance has been increased more and more.

Up to now, the amino acid analyzer utilizing high pressure liquid chromatography is only available as practical method for measuring amino acids. Individual amino acids may be measured by the method, but the instrument is expensive and there is the disadvantage that the measurement requires a long time. Therefore, such method is hardly available in clinical hospitals where screening tests are carried out every day, but is available exclusively in research institute.

Several methods for measuring amino acid utilizing enzymatic reaction are known. For example, there are known ① the method by coupling leucine dehydrogenase to branched-chain amino acid in the presence of nicotinamide adenine dinucleotide (abbreviated as "NAD" hereafter), and then measuring resulting reduced-NAD at 340 nm, ② the method by coupling decarboxylase to individual amino acid and then measuring either resulting carbon dioxide gas manometrically or resulting monoamine by coloration, and ③ the method by coupling amino acid oxidase with amino acid, and then measuring resulting hydrogen peroxide [These methods are described in Methods of Enzymatic Analysis, 2nd Edition, 4, 1662 and 1669 (1974) and ibid, 3rd Edition, 8, 318 (1985), both published by Academic Press.], and further ④ the method by coupling phenylalanine ammonialiase with phenylalaine and tyrosine to convert into cinnamate and coumarate, respectively, and then measuring them at 290 ~ 315 nm [described in Science, 197, 665 (1977)].

However, there are disadvantages in these methods. That is to say, Method ① and Method ④ are highly affected by ultraviolet absorption of proteins in sample and they, therefore, have poor accuracy. Further, sensitivity of detection is insufficient for practical use. Method ②, i.e. manometrical measurement, has good sensitivity, but requires special instrument for measurement and a tedious procedure. The enzyme used in Method ③ has no specificity for various amino acids and it is impossible to measure selectively aromatic amino acids and branched-chain amino acids.

As mentioned above, several methods for measurement utilizing enzymatic reaction are proposed, but they have many problems to be solved before practical use.

### 3. Means to solve the problem

Research and development have been carried out by the present inventors in order to establish a method for measuring selectively and rapidly phenylalanine and/or tyrosine and branched-chain amino acids with accuracy and range suitable for practical use. As a result of energetic investigation, it is found that the purpose has accomplished by coupling enzymes which specifically react with (i.e. enzymes being highly specific for) or phenylalanine and/or tyrosine to branched-chain amino acids, and then introducing into the system for detection of hydrogen peroxide, having completed this invention.

Further research has revealed that the ratio of phenylalanine to branched-chain amino acids or the ratio of tyrosine to branched-chain amino acids, in blood is fully correlated with Fischer's ratio, and therefore, it may be used for judgement of the degree of liver damage and for monitor of amino acid in the therapy by amino acids-infusion, instead of Fischer's ratio.

The method for the measurement, of the present invention has never been tried and has confirmed by our experiments for the first time.

## SUMMARY OF THE INVENTION

Accordingly, an object of the present invention is to provide new methods for selective measurement of the amino acids mentioned above.

## BRIEF DESCRIPTION OF THE DRAWINGS

In the drawings:

Figure 1 is a calibration curve of the aromatic amino acids phenylalanine and/or tyrosine measured by the method of the present invention.

Figure 2 is a calibration curve of branched-chain amino acids, measured by the method of the present invention.

Figure 3 is a graph showing the correlation between the ratio of tyrosine to branched-chain amino acids, determined by the method of the present invention and Fischer's ratio.

## DETAILED DESCRIPTION OF THE INVENTION

### 1. Constitution of the Invention

The present invention is concerned with methods for selective measurement of phenylalanine and/or tyrosine or branched-chain amino acids, which are characterized by coupling phenylalanine-4-monooxygenase and tyrosine decarboxylase, and/or phenylalanine decarboxylase and/or tyrosine decarboxylase specific for phenylalanine and/or tyrosine, and then by coupling tyramine oxidase to produce hydrogen peroxide or coupling leucin dehydrogenase specific for branched-chain amino acids, and then by coupling lactic acid dehydrogenase and lactic acid oxidase to produce hydrogen peroxide, and then measuring the said each hydrogen peroxide.

In the present invention, examples of branched-chain amino acid are valine, leucine and isoleucine. Any test samples containing the above amino acids are available for this invention, and for example, samples in body fluid, such as serum, plasma and urine and infusion of amino acids are available. In this invention, an operation of removing proteins from a sample is unnecessary, but the sample from which a protein is removed by using sulfosalicylic acid, trichloroacetic acid, picric acid, perchloric acid or wolframic acid and then of which pH is neutralized again, is also available for test sample.

In general terms, the present invention is concerned with (1) (a) methods for the measurement of phenylalanine and tyrosine in a sample which are characterized by coupling the enzyme specific for tyrosine and phenylalanine or coupling successively the enzyme specific for the above individual aromatic amino acids, and then introducing the product into a system for the evolution of hydrogen peroxide to measure the resulting hydrogen peroxide, or (b) methods for the measurement of phenylalanine or tyrosine in a sample which are characterized by coupling the enzyme specific for phenylalanine or tyrosine alone, and then introducing the product into a system for the evolution of hydrogen peroxide to measure the resulting hydrogen peroxide; or (2) methods for the measurement of all branched-chain amino acids in a sample which are characterized by coupling the enzyme specific for all branched-chain amino acids, i.e. valine, leucine and isoleucine or coupling successively the enzyme specific for individual branched-chain amino acids, and then introducing the product into a system for the evolution of hydrogen peroxide to measure the resulting hydrogen peroxide.

The following typical enzymatic reactions illustrate, but do not limit, the present invention in detail.

The methods for the measurement of phenylalanine and/or tyrosine are shown in the following scheme.

Scheme on Measurement of Phenylalanine and/or Tyrosine

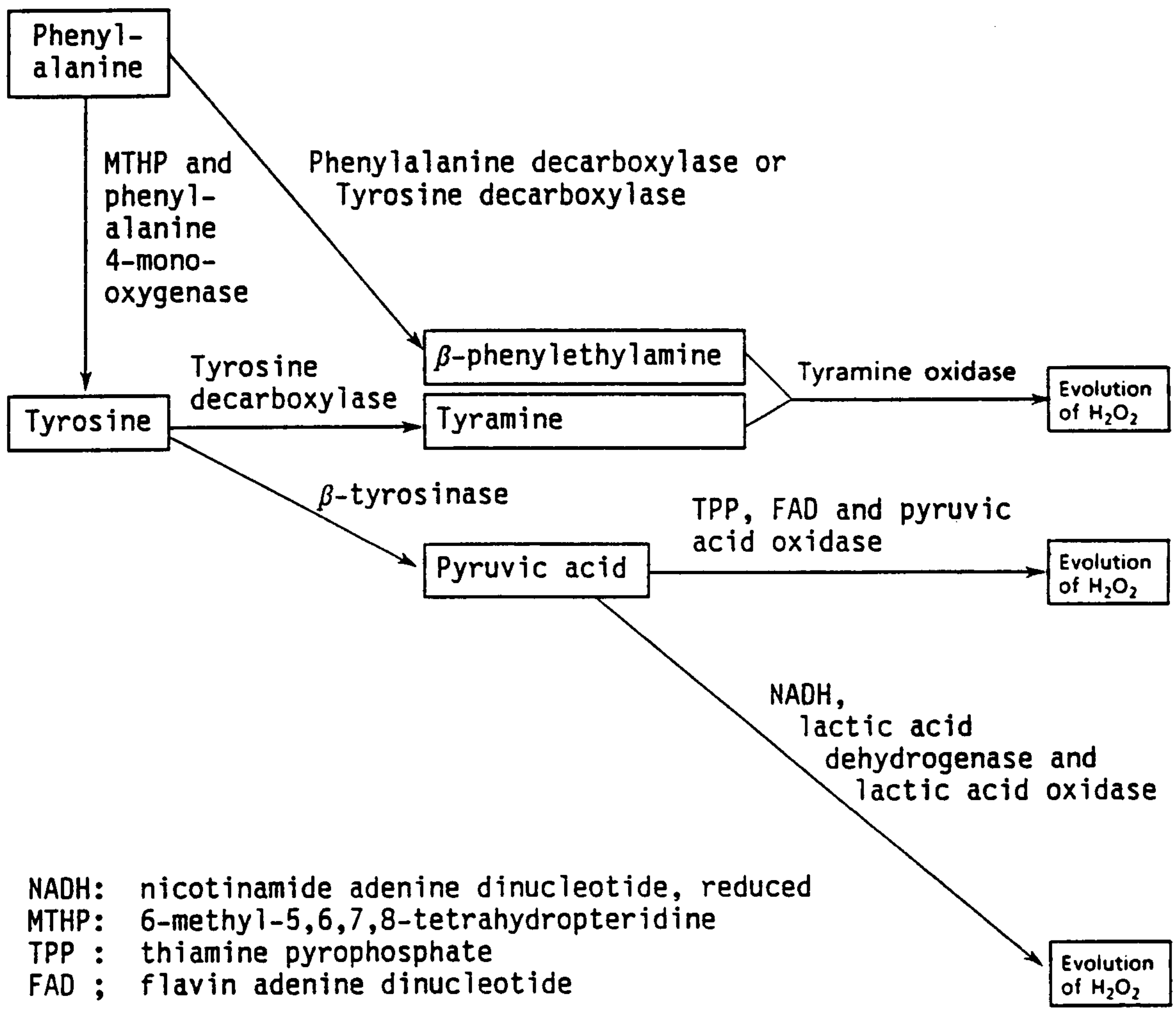


Examples of enzymes being specific for phenylalanine are phenylalanine decarboxylase and phenylalanine 4-monooxygenase. Enzymes being specific for tyrosine are tyrosine decarboxylase and β-tyrosinase. Furthermore, tyrosine decarboxylase in the high concentration is known to couple with phenylalanine.

Enzymes which derive from any organisms may be used in the present invention. For example, phenylalanine decarboxylase can be prepared from Streptococcus faecalis, and phenylalanine 4-monooxygenase can be extracted and purified from liver of rats [cf. J. Biol. Chem. 245, 4745 (1970)] and Pseudomonas [cf. J. Biol. Chem. 250, 6672 (1975)]. This 4-monooxygenase catalyses the quantative conversion of phenylalanine into tyrosine in the presence of 6-methyl-5,6,7,8-tetrahydropteridine. As tyrosine decarboxylase, apoenzyme of tyrosine decarboxylase which is prepared by crushing and extracting from acetone-dried bacterium of Streptococcus faecalis and then purifying by molecular sieve, may be used. The enzyme catalyses the conversion of tyrosine into tyramine in the presence of pyridoxal 5-phosphate. When holoenzyme of tyrosine decarboxylase is used, pyridoxal 5-phosphate is unnecessary. β-tyrosinase may be purified from Escherichia intermedia [cf. J. Biol. Chem., 245, 1767 and 1773 (1970)].

By reacting these enzymes with any combination phenylalanine and tyrosine in a sample may be converted into β-phenylethylamine, tyramine and/or pyruvic acid, or by reacting these enzymes alone, phenylalanine or tyrosine in a sample may be converted into β-phenylethylamine, tyramine or pyruvic acid. Preferably, ① phenylalanine and tyrosine may be converted into β-phenylethylamine and tyramine, respectively, by coupling 0.1 ~ 5 U/mℓ (it means the final concentration, and does the same hereinafter.) of phenylalanine decarboxylase and 0.1 ~ 2 U/mℓ of tyrosine decarboxylase (or 0.1 ~ 2 U/mℓ of its

apoenzyme and 1 μM ~ 1 mM of pyridoxal 5-phosphate), ② both phenylalanine and tyrosine may be converted into tyramine by coupling 0.1 ~ 5 U/mℓ of phenylalanine 4-monooxygenase (in the presence of 10 μM ~ 1 mM of 6-methyl-5,6,7,8-tetrahydropteridine) and 0.1 ~ 2 U/mℓ of tyrosine decarboxylase (or its apoenzyme and pyridoxal 5-phosphate hereinbefore described), ③ both phenylalanine and tyrosine may be converted into pyruvic acid by coupling 0.1 ~ 5 U/mℓ of phenylalanine 4-monooxygenase (in the presence of 10 μM ~ 1 mM of 6-methyl-5,6,7,8-tetrahydropteridine) and 0.1 ~ 5 U/mℓ of β-tyrosinase, ④ phenylalanine as well as tyrosine may be converted into β-phenylethylamine and tyramine, respectively, at the same time, by coupling 10 ~ 50 times of tyrosine decarboxylase used in the case ① (or 10 ~ 50 times of its apoenzyme), preferably the final concentration being 1 ~ 20 U/mℓ, ⑤ phenylalanine may be converted into β-phenylethylamine by coupling 0.1 ~ 5 U/mℓ of phenylalanine decarboxylase, or ⑥ tyrosine may be converted into tyramine or pyruvic acid by coupling 0.1 ~ 2 U/mℓ of tyrosine decarboxylase (or its apoenzyme and pyridoxal 5-phosphate hereinbefore described) or by coupling 0.1 ~ 5 U/mℓ of β-tyrosinase, respectively. The above methods ① to ④ are those for simultaneous measurement of all both phenylalanine and tyrosine in sample, and on the other hand, the method ⑤ is that for measurement of phenylalanine only and the method ⑥ is that for measurement of tyrosine only. The method ④ and ⑥ are more preferable at the viewpoint of economy and simplicity.

The introduction of the metabolites of aromatic amino acids into a system for the evolution of hydrogen peroxide may be carried out, for example, by coupling tyramine oxidase to β-phenylethylamine and/or tyramine (Tyramine oxidase may react with β-phenylethylamine as well as tyramine to produce hydrogen peroxide.), by coupling pyruvic acid oxidase to pyruvic acid in the presence of thiamine pyrophosphate and flavin adenine dinucleotide, and by coupling lactic acid dehydrogenase and lactic acid oxidase to pyruvic acid in the presence of NADH (i.e. nicotinamide adenine dinucleotide, reduced), and the like. Any enzymes and reagents used for the said reactions are on the market.

Preferably, 0.1 ~ 5 U/mℓ of tyramine oxidase, or 1 ~ 20 U/mℓ of pyruvic acid oxidase, 0.1 ~ 1 mM of thiamine pyrophosphate and 1 μM ~ 0.1 mM of flavin adenine dinucleotide, or 1 ~ 20 U/mℓ of lactic acid dehydrogenase, 0.1 ~ 5 U/mℓ of lactic acid oxidase and 0.1 ~ 1 mM of NADH may be practically used.

Preferred methods of the present invention for measuring phenylalanine and tyrosine, i.e. to the amino acids are as follows:

(1) method by coupling, to the amino acids, phenylalanine decarboxylase and tyrosine decarboxylase simultaneously, and then by coupling tyramine oxidase,
(2) method by coupling, to the amino acids, phenylalanine 4-monooxygenase in the presence of 6-methyl-5,6,7,8-tetrahydropteridine and tyrosine decarboxylase simultaneously, and then by coupling tyramine oxidase,
(3) method by coupling, to the amino acids, phenylalanine 4-monooxygenase in the presence of 6-methyl-5,6,7,8-tetrahydropteridine and β-tyrosinase simultaneously, and then by coupling pyruvic acid oxidase in the presence of thiamine pyrophosphate and flavin adenine dinucleotide,
(4) method by coupling, to the amino acids, phenylalanine 4-monooxygenase in the presence of 6-methyl-5,6,7,8-tetrahydropteridine and β-tyrosinase simultaneously, and then by coupling lactic acid dehydrogenase and lactic acid oxidase simultaneously in the presence of nicotinamide adenine dinucleotide, reduced,
(5) method by coupling, to the amino acids, tyrosine decarboxylase in the final concentration of 1 ~ 20 U/mℓ, and then by coupling tyramine oxidase,
(6) method by coupling, to phenylalanine, phenylalanine decarboxylase and then by coupling tyramine oxidase,
(7) method by coupling, to tyrosine, tyrosine decarboxylase and then by coupling tyramine oxidase,
(8) method by coupling, to tyrosine, β-tyrosinase and then by coupling pyruvic acid oxidase in the presence of thiamine pyrophosphate and flavin adenine dinucleotide, and
(9) method by coupling, to tyrosine β-tyrosinase, and then by coupling lactic acid dehydrogenase and lactic acid oxidase simultaneously in the presence of nicotinamide adenine dinucleotide, reduced.

More preferably, the method of the present invention may be carried out by coupling, to the amino acids, tyrosine decarboxylase in the final concentration of 1 ~ 20 U/mℓ, and then by coupling, to the resulting tyramine and β-phenylethylamine, tyramine oxidase to evolute hydrogen peroxide; and may be carried out by coupling to tyrosine, tyrosine decarboxylase and then by coupling to the resulting tyramine, tyramine oxidase to evolute hydrogen peroxide.

Resulting hydrogen peroxide may be measured by using a known system for the detection of hydrogen peroxide. As a representative system, the measurement may be carried out in the presence of peroxidase, by using, as an origin of color, a compound which is prepared by oxidatively condensing 4-aminoantipyrine or 3-methyl-2-benzothiazolinone-hydrazone•hydrochloride, with a derivative of aniline, anisidine or toluidine

[e.g. N-ethyl-H-(2-hydroxy-3-sulfopropyl)-m-toluidine (abbreviated as "TOOS", hereafter)]. The enzymatic reactions may be carried out by known methods.

Practically, the measurement of the present invention may be carried out by adding a test sample containing phenylalanine and or tyrosine and an adequate volume of water, to an enzyme solution which comprises enzymes specific for phenylalanine and/or tyrosine, enzymes for introducing metabolites produced by the action of the said enzymes into a system for the evolution of hydrogen peroxide, and enzymes for detecting the said hydrogen peroxide, and of which pH is adjusted to 4 ~ 8, preferably 5.5 ~ 6 by buffer solutions, to obtain a definite volume of mixture solution, by reacting the mixture for 5 ~ 10 minutes at 37 ° C, and then by measuring by spectrophotometer.

If necessary, the enzyme solutions may be divided into some groups to add successively.

As buffer solution used in the above reaction, any buffer solution being adjustable to pH 4 ~ 8 may be available, for example, acetic acid buffer solution, citric acid buffer Solution, Sørensen buffer solution, McIlvaine buffer solution, Good buffer solution etc, preferably McIlvaine buffer solution. The buffer solution may be used in the range of 10 ~ 200 mM.

Next, the methods for the measurement of branched-chain amino acids are shown in the following scheme.

Scheme on Measurement of Branched-Chain Amino Acids

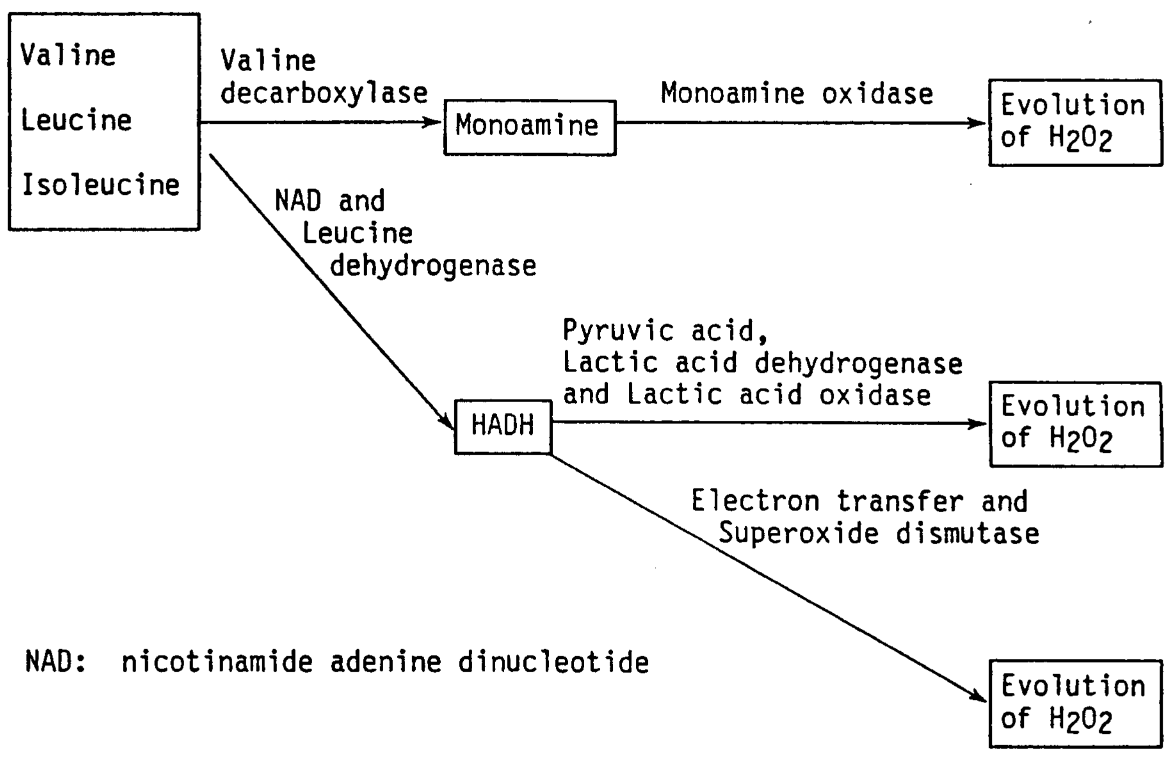


Examples of enzyme specific for branched-chain amino acid are valine decarboxylase (alias leucine decarboxylase) and leucine dehydrogenase, both being specific for all branched-chain amino acids, i.e. valine, leucine and isoleucine.

Enzymes which derive from any organisms may be used in the present invention. For example, valine decarboxylase can be purified from Proteus vulgaris [cf. J. Ger. Microbiol. 17, 602 (1957)] and leucine dehydrogenase can be purified from Bacillus sphaericus [cf. J. Biol. Chem. 253, 5719 (1978)]. Valine decarboxylase (or its apoenzyme in the presence of pyridoxal 5-phosphate) catalyses the quantitative conversion of branched-chain amino acids into monoamine. Leucine dehydrogenase acts branched-chain amino acids to convert quantatively coupled NAD into NADH.

Practically, 0.1 ~ 5 U/mℓ of valine decarboxylase (or its apoenzyme in the presence of 1 µM ~ 1 mM of pyridoxal 5-phosphate), or 1 ~ 20 U/mℓ of leucine dehydrogenase and 1 ~ 20 mM of NAD may be suitably

used.

The introduction of the resulting monoamine or NADH into a system for the evolution of hydrogen peroxide may be carried out by coupling monoamine oxidase to monoamine, by coupling lactic acid dehydrogenase and lactic acid oxidase to NADH in the presence of pyruvic acid, or by coupling superoxide dismutase to NADH in the presence of electoron carrier (e.g. phenazine methosulfate, 1-methoxyphenazine methosulfate, 9-dimethylaminobenzo-α-phenazoxonium chloride etc.) [cf. Jap. J. Clin. Chem., 15, 20 (1986)]. Any enzymes and reagents used for the said reactions are on the market. The enzymatic reactions are well-known per se, and may be carried out by known methods.

Practically, 0.5 ~ 10 U/mℓ of monoamine oxidase, or 1 ~ 20 U/mℓ of lactic acid dehydrogenase, 0.1 ~ 5 U/mℓ of lactic acid oxidase and 0.1 ~ 5 mM of pyruvic acid, or 10 ~ 100 U/mℓ of superoxide dismutase and 1 ~ 100 μM of phenazine methosulfate may be suitably used.

Preferred methods for measuring branched-chain amino acids, of the present invention, are as follows:

(1) method by coupling, to all branched-chain amino acids, valine decarboxylase and then by coupling monoamine oxidase,

(2) method by coupling, to all branched-chain amino acids, leucine dehydrogenase in the presence of nicotinamide adenine dinucleotide, and then by coupling lactic acid dehydrogenase and lactic acid oxidase simultaneously in the presence of pyruvic acid, and

(3) method by coupling, to all branched-chain amino acids, leucine dehydrogenase in the presence of nicotinamide adenine dinucleotide, and then by coupling superoxide dismutase in the presence of electron carrier.

More preferably, the method of the present invention may be carried out by coupling leucine dehydrogenase to branched-chain amino acids in the presence of NAD, and then coupling lactic acid dehydrogenase and lactic acid oxidase to the resulting NADH in the presence of pyruvic acid, to evolute hydrogen peroxide.

Resulting hydrogen peroxide may be measured by using the known system for the detection of hydrogen peroxide, hereinbefore described.

Practically, the measurement of the present invention may be carried out by adding a test sample containing branched-chain amino acids and an adequate volume of water, to an enzyme solution which comprises enzymes specific for branched-chain amino acids, enzymes for introducing metabolites produced by the action of the said enzymes into a system for the evolution of hydrogen peroxide, and enzymes for detecting the said hydrogen peroxide, and of which pH is adjusted to 6 ~ 11, preferably pH 6 ~ 8 in using valine decarboxylase and pH 7.5 ~ 9.5 in using leucine dehydrogenase, by buffer solution, to obtain a definite volume of mixture solution, by reacting the mixture for 5 ~ 10 minutes at 37°C, and then by measuring by spectrophotometer. If necessary, the enzyme solutions may be divided into some groups to add successively.

As buffer solution used in the above reaction, any buffer solution being adjustable to pH 6 ~ 11 may be available, for example, Sørensen buffer solution, McIlvaine buffer solution, tris(hydroxymethyl)aminomethane (Tris) buffer solution, Good buffer solution, carbonic acid buffer solution etc, preferably Tris buffer solution. The buffer solution may be used the range of 10 ~ 200 mM.

The selective measurement of phenylalanine and/or tyrosine or branched-chain amino acids, of the present invention may be carried out, manually by using a spectrophotometer or automatically by using a autoanalyzer for biochemical analysis. More preferably, it may be carried out by using a autoanalyzer for biochemical analysis, which may enable to measure optical density at two points for determinating aromatic amino acids and branched-chain amino acids, respectively. In both method, the system for detecting hydrogen peroxide in measuring phenylalanine and/or tyrosine is preferable the same as that in measuring branched-chain amino acids.

Furthermore, when using a autoanalyzer for biochemical analysis, a pre-treating system for removing interfering substances may be preferably incorporated. For example, in order to remove interfering substance which produces hydrogen peroxide, a test sample should be introduced into a pre-treating system comprising peroxidase and TOOS, and in order to remove interfering substance which produces NADH in the presence of NAD, a test sample should be introduced into a pre-treating system comprising NAD and an electron carrier, e.g. 1-methoxyphenazine methosulfate, and in order to remove lactic acid as a interfering substance, a test sample should be introduced into a pre-treating system comprising lactic acid oxidase, TOOS and peroxidase.

From amounts of phenylalanine and/or tyrosine and branched-chain amino acids, measured as described hereinbefore, Fischer's ratio or the ratio of phenylalanine or tyrosine, to all branched-chain amino acids can be calculated, and therefore can be utilized in judgement of the degree of liver damage and in monitor of amino acids in the therapy by amino acids-infusion.

Furthermore, applying the methods of the present invention, a simplified method by absorbing and fixing the desired enzymes and reagents to a solid phase such as paper etc, and then applying a solution of test sample thereon to react enzymatically may also be clinically available as semi-quantative analysis.

## 2. Examples

The following examples illustrate, but do not limit, the present invention in detail.

### Example 1

Measurement of aromatic amino acids, viz. phenylalanine and/or tyrosine

To an enzyme solution comprising 50 U/mℓ of apoenzyme of tyrosine decarboxylase (120 μℓ), 100 μM of pyridoxal 5-phosphate (50 μℓ), 10 U/mℓ of tyramine oxidase (100 μℓ), 20 U/mℓ of peroxidase (100 μℓ), 50 mM of TOOS (60 μℓ) and 3 mM of 4-aminoantipyrine (100 μℓ), and adjusting to pH 6 by adding with 0.1 M of McIlvaine buffer solution (pH 6.0, 300 μℓ), were added 50 μℓ of a test sample containing mixtures of various proportions of phenylalanie and/or tyrosine, as shown in the following table and further added an appropriate amount of water, to obtain a total volume of 1 mℓ of a mixed solution. The obtained mixture was reacted for 5 minutes at 37° C, and then the color intensity was measured at 546 nm by spectrophotometer. The result is shown in Figure 1, wherein the intercept of y-axis shows the color intensity of water as control.

As understood from the figure, nearly straight calibration curve was given for test samples containing mixtures of various proportions of phenylalanine and/or tyrosine. Therefore, it is understood that a total amount of the each aromatic amino acids can be quantatively measured for test samples containing mixture of any proportions of phenylalanine and tyrosine.

| Proportions of aromatic amino acids in test samples | | |
|---|---|---|
| Tyrosine (μM) | Phenylalanine (μM) | Total (μM) |
| 0 | 0 | 0 |
| 200 | 0 | 200 |
| 0 | 400 | 400 |
| 200 | 400 | 600 |
| 600 | 200 | 800 |
| 400 | 600 | 1000 |

### Example 2

Measurement of branched-chain amino acids

To an enzyme solution comprising 80 U/mℓ of leucine dehydrogenase (100 μℓ), 40 mM of NAD (100 μℓ), 100 U/mℓ of lactic acid dehydrogenase (50 μℓ), 50 U/mℓ of lactic acid oxidase (50 μℓ), 10 mM of pyruvic acid (100 μℓ), 20 U/mℓ of peroxidase (100 μℓ), 50 mM of TOOS (100 μℓ) and 1 mM of 4-aminoantipyrine (100 μℓ), and adjusting to pH 7.5 by adding with 0.2 M of tris(hydroxymethyl)-aminomethane buffer solution (pH 7.5, 150 μℓ), were added 20 μℓ of a test sample containing mixtures of various proportions of branched-chain amino acids, as shown in the following table and further added an appropriate amount of water, to obtain a total volume of 1 mℓ of a mixed solution. The obtained mixture was reacted for 5 minutes at 37° C, and then the color intensity was measured at 546 nm by spectrophotometer. The result is shown in Figure 2, wherein the intercept of y-axis shows the color intensity of water as control.

As understood from the figure, nearly straight calibration curve was given for test samples containing mixtures of various proportions of branched-chain amino acids. Therefore, it is understood that a total amount of branched-chain amino acids can be quantatively measured for test samples containing mixture of any proportions of branched-chain amino acids.

| Proportions of branched-chain amino acids in test samples | | | |
|---|---|---|---|
| Valine (μM) | Leucine (μM) | Isoleucine (μM) | Total (μM) |
| 0 | 100 | 0 | 100 |
| 0 | 0 | 200 | 200 |
| 300 | 0 | 0 | 300 |
| 200 | 100 | 100 | 400 |
| 100 | 200 | 200 | 500 |
| 100 | 300 | 200 | 600 |
| 200 | 200 | 300 | 700 |
| 100 | 400 | 300 | 800 |
| 300 | 200 | 400 | 900 |
| 400 | 400 | 200 | 1000 |

Example 3

Measurement of phenylalanine and/or tyrosine in serum

By using serum (50 μℓ) containing or not containing mixtures of various proportions of phenylalanine and/or tyrosine, instead of test samples used in Example 1, phenylalanine and/or tyrosine were measured by the same procedure as described in Example 1, and the recovery of addition was calculated. The result is shown in the following table.

| Added | | | Measured (μM) | Recovery ratio (%) |
|---|---|---|---|---|
| Tyrosine (μM) | Phenylalanine (μM) | Total (μM) | | |
| 0 | 0 | 0 | 164.3 | |
| 100 | 0 | 100 | 266.9 | 102.6 |
| 0 | 200 | 200 | 374.3 | 105.0 |
| 200 | 200 | 400 | 577.2 | 103.2 |

Good results, i.e. 102 ~ 105% in the recovery of addition were given. From the above fact, it is understood that it is possible to measure well exactly without the influence of other components in serum even when using serum as sample.

Example 4

Measurement of tyrosine and branched-chain amino acids in plasma

(1) Measurement of tyrosine

0.1 M of McIlvaine buffer solution (pH 6.0, 100 mℓ) containing 250 U of peroxidase and 0.375 mmole of TOOS was prepared as "the 1st reagent". Further, 0.1 M of McIlvaine buffer solution (pH 6.0, 25 mℓ) containing 56.3 U of apoenzyme of tyrosine decarboxylase, 1.25 μmole of pyridoxal 5-phosphate, 125 U of tyramine oxidase and 0.038 mmole of 4-aminoantipyrine was prepared as "the 2nd reagent".

The measurement was carried out with a autoanalyzer for biochemical analysis (Hitachi, Model 705), using 10 μℓ of plasma as sample, 400 μℓ of the 1st reagent and 100 μℓ of the 2nd reagent. The 1st reagent was added to a sample, and the mixture was reacted for 5 minutes at 37°C, and then after addition of the 2nd reagent thereto, the obtained mixture was reacted for further 5 minutes at 37°C. The color

intensity was measured at 546 nm by endpoint method.

(2) Measurement of branched-chain amino acids

50 mM of Tris buffer solution (pH 7.5, 100 mℓ) containing 313 U of lactic acid oxidase, 0.125 mM of TOOS and 250 U of peroxidase was prepared as "the 1st reagent'. Further, 50 mM of Tris buffer solution (pH 7.5, 25 mℓ) containing 938 U of leucine dehydrogenase, 0.5 mmole of NAD, 0.13 mmole of pyruvic acid, 625 U of lactic acid dehydrogenase, and 0.038 mmole of 4-aminoantripyrine was prepared as "the 2nd reagent".

The measurement was carried out by the same procedure as above-described for tyrosine (provided that 5 μℓ of plasma was used as sample).

(3) Correlation between the ratio of tyrosine to branched-chain amino acids, determined by the above method (present invention) and Fischer's ratio determined by using an amino acid analyzer (comparison)

Tyrosine and branched-chain amino acids were measured by the above methods, by using 67 test samples (human plasma) and then the ratio (branched-chain amino acids/tyrosine) was determined. Next, individual amino acids were measured by using the same 67 test samples, with an amino acid analyzer (Hitachi, Model 835; To plasma was added the same volume of 10% sulfosalicylic acid and the mixture was allowed to stand for 30 minutes. After centrifuging the mixture, the obtained supernatant was used as test sample. The detection was carried out by labeling with o-phthalaldehyde and measuring at 348 nm for excitation and 450 nm for emission by fluorescence detector.) and then Fischer's ratio (branched-chain amino acids/tyrosine + phenylalanine) was determined. The correlation between the ratio of tyrosine to branched-chain amino acids, determined by the method of the present invention and Fischer's ratio is shown in Figure 3.

$Y = 2.123X - 0.222$

correlation coefficient: $y = 0.936$

These value shows that the correlation between two ratios is excellent. Therefore, it is understood that the ratio of tyrosine to branched-chain amino acids, instead of Fischer's ratio, may be used for the purpose of the invention.

3. Effect

The present invention relates to excellent methods for the measurement of amino acids, which enable to measure selectively and rapidly the aromatic amino acids phenylalanine and/or tyrosine and branched-chain amino acids with accuracy and range suitable for practical use. By using the present methods, it is possible to measure the ratio of all or individual of the said aromatic amino acids to branched-chain amino acids in a sample, and it can be utilized in judgement of the degree of liver damage and in monitor of amino acid in the therapy by amino acids-infusion.

**Claims**

**1.** A method of selective measurement of phenylalanine and/or tyrosine or branched chain amino acid, which is characterized by coupling phenylalanine-4-monooxygenase and tyrosine decarboxylase, and/or phenylalanine decarboxylase and/or tyrosine decarboxylase specific for phenylalanine and/or tyrosine, and then by coupling tyramine oxidase to produce hydrogen peroxide or coupling leucin dehydrogenase specific for branched-chain amino acids, and then by coupling lactic acid dehydrogenase and lactic acid oxidase to produce hydrogen peroxide, and then measuring the said each hydrogen peroxide.

**2.** A method for selective measurement according to claim 1, which is one for the measurement of phenylalanine and tyrosine in a sample, which is characterized by coupling tyrosine decarboxylase specific for phenylalanine and tyrosine, or coupling successively phenylalanine-4-monooxygenase and tyrosine decarboxylase, or phenylalanine decarboxylase and tyrosine decarboxylase specifc for phenylalanine or tyrosine, and then by coupling tyramine oxidase to produce hydrogen peroxide to measure the resulting hydrogen peroxide.

3. A method for selective measurement according to claim 1, which is one for the measurement of phenylalanine or tyrosine in a sample, which is characterized by coupling phenylalanine decarboxylase or tyrosine decarboxylase specific for phenylalanine or tyrosine alone, and then by coupling tyramine oxidase to produce hydrogen peroxide to measure the resulting hydrogen peroxide.

4. A method for selective measurement according to claim 2, which is one for the measurement of phenylalanine and tyrosine in a sample, which is characterized by coupling, to phenylalanine and tyrosine, phenylalanine decarboxylase and tyrosine decarboxylase simultaneously, and tyramine oxidase, successively, and then measuring the resulting hydrogen peroxide.

5. A method for selective measurement according to claim 2, which is one for the measurement of phenylalanine and tyrosine in a sample, which is characterized by coupling, to phenylalanine and tyrosine, phenylalanine 4-monooxygenase in the presence of 6-methyl-5,6,7,8-tetrahydropteridine and tyrosine decarboxylase simultaneously, and tyramine oxidase, successively, and then measuring the resulting hydrogen peroxide.

6. A method for selective measurement according to claim 2, which is one for the measurement of phenylalanine and tyrosine in a sample, which is characterized by coupling, to phenylalanine and tyrosine, tyrosine decarboxylase in the final concentration of 1 ~ 20 U/mℓ and tyramine oxidase, successively, and then measuring the resulting hydrogen peroxide.

7. A method for selective measurement according to claim 3, which is one for the measurement of phenylalanine in a sample, which is characterized by coupling to phenylalanine, phenylalanine decarboxylase, and tyramine oxidase, successively, and then measuring the resulting hydrogen peroxide.

8. A method for selective measurement according to claim 3, which is one for the measurement of tyrosine in a sample, which is characterized by coupling to tyrosine, tyrosine decarboxylase and tyramine oxidase, successively, and then measuring the resulting hydrogen peroxide.

## Revendications

1. Méthode de mesure sélective de la phénylalanine et/ou de la tyrosine ou d'un aminoacide à chaîne ramifiée, qui est caractérisée en ce qu'on couple de la phénylalanine-4-monooxygénase et de la tyrosine décarboxylase, et/ou de la phénylalanine décarboxylase et/ou de la tyrosine décarboxylase spécifiques pour la phénylalanine et/ou la tyrosine, et en ce qu'on couple ensuite de la tyramine oxydase pour produire du peroxyde d'hydrogène, ou bien en ce qu'on couple de la leucine déshydrogénase spécifique pour les aminoacides à chaîne ramifiée, et en ce qu'on couple ensuite l'acide lactique déshydrogénase et l'acide lactique oxydase pour produire du peroxyde d'hydrogène, et en ce qu'on mesure ensuite dans chaque cas ledit peroxyde d'hydrogène.

2. Méthode de mesure sélective selon la revendication 1, qui est une méthode de mesure de la phénylalanine et de la tyrosine dans un échantillon, et qui est caractérisée en ce qu'on couple de la tyrosine décarboxylase spécifique pour la phénylalanine et la tyrosine, ou en ce qu'on couple successivement de la phénylalanine-4-monooxygénase et de la tyrosine décarboxylase, ou de la phénylalanine décarboxylase et de la tyrosine décarboxylase spécifiques pour la phénylalanine ou la tyrosine, et en ce qu on couple ensuite de la tyramine oxydase pour produire du peroxyde d'hydrogène afin de mesurer le peroxyde d'hydrogène produit.

3. Méthode de mesure sélective selon la revendication 1, qui est une méthode de mesure de la phénylalanine ou de la tyrosine dans un échantillon, et qui est caractérisée en ce qu'on couple de la phénylalanine décarboxylase ou de la tyrosine décarboxylase spécifiques pour la phénylalanine ou la tyrosine seules, et en ce qu'on couple ensuite de la tyramine oxydase pour produire du peroxyde d'hydrogène afin de mesurer le peroxyde d'hydrogène formé.

4. Méthode de mesure sélective selon la revendication 2, qui est une méthode de mesure de la phénylalanine et de la tyrosine dans un échantillon, et qui est caractérisée en ce qu'on couple simultanément, à la phénylalanine et à la tyrosine, de la phénylalanine décarboxylase et de la tyrosine décarboxylase, et successivement de la tyramine oxydase, et en ce qu'on mesure ensuite le peroxyde

d'hydrogène formé.

5. Méthode de mesure sélective selon la revendication 2, qui est une méthode de mesure de la phénylalanine et de la tyrosine dans un échantillon, et qui est caractérisée en ce qu'on couple simultanément, à la phénylalanine et à la tyrosine, de la phénylalanine-4-monooxygénase en présence de 6-méthyl-5,6,7,8-tétrahydroptéridine et de la tyrosine décarboxylase, et successivement de la tyramine oxydase, et en ce qu'on mesure ensuite le peroxyde d'hydrogène formé.

6. Méthode de mesure sélective selon la revendication 2, qui est une méthode de mesure de la phénylalanine et de la tyrosine dans un échantillon, et qui est caractérisée en ce qu'on couple successivement, à la phénylalanine et à la tyrosine, de la tyrosine décarboxylase en une concentration finale de 1-20 U/ml, et de la tyramine oxydase, et en ce qu'on mesure ensuite le peroxyde d'hydrogène formé.

7. Méthode de mesure sélective selon la revendication 3, qui est une méthode de mesure de la phénylalanine dans un échantillon, et qui est caractérisée en ce qu'on couple successivement, à la phénylalanine, de la phénylalanine décarboxylase et de la tyramine oxydase, et en ce qu'on mesure ensuite le peroxyde d'hydrogène formé.

8. Méthode de mesure sélective selon la revendication 3, qui est une méthode de mesure de la tyrosine dans un échantillon, et qui est caractérisée en ce qu'on couple successivement, à la tyrosine, de la tyrosine décarboxylase et de la tyramine oxydase, et en ce qu'on mesure ensuite le peroxyde d'hydrogène formé.

## Patentansprüche

1. Verfahren zur selektiven Bestimmung von Phenylalanin und/oder Tyrosin oder einer verzweigtkettigen Aminosäure durch Kuppeln von Phenylalanin-4-monooxygenase und Tyrosindecarboxylase und/oder für Phenylalanin und/oder Tyrosin spezifische Phenylalanindecarboxylase und/oder Tyrosindecarboxylase und anschließendes Kuppeln von Tyraminoxidase zur Bildung von Wasserstoffperoxid oder durch Kuppeln von für verzweigtkettige Aminosäuren spezifischer Leucindehydrogenase und anschließendes Kuppeln von Milchsäuredehydrogenase und Milchsäureoxidase zur Bildung von Wasserstoffperoxid und anschließendes Ausmessen des jeweiligen Wasserstoffperoxids.

2. Verfahren zur selektiven Bestimmung nach Anspruch 1 zur Ermittlung von Phenylalanin und Tyrosin in einer Probe durch Kuppeln von für Phenylalanin und Tyrosin spezifischer Tyrosindecarboxylase oder durch sukzessives Kuppeln von Phenylalanin-4-monooxygenase und Tyrosindecarboxylase oder von für Phenylalanin oder Tyrosin spezifischer Phenylalanindecarboxylase und Tyrosindecarboxylase und anschließendes Kuppeln von Tyraminoxidase zur Bildung von Wasserstoffperoxid zur Bestimmung des gebildeten Wasserstoffperoxids.

3. Verfahren zur selektiven Bestimmung nach Anspruch 1 zur Ermittlung von Phenylalanin oder Tyrosin in einer Probe durch Kuppeln von für Phenylalanin oder Tyrosin alleine spezifischer Phenylalanindecarboxylase oder Tyrosindecarboxylase und anschließendes Kuppeln von Tyraminoxidase zur Bildung von Wasserstoffperoxid zur Bestimmung des gebildeten Wasserstoffperoxids.

4. Verfahren zur selektiven Bestimmung nach Anspruch 2 zur Ermittlung von Phenylalanin und Tyrosin in einer Probe durch gleichzeitiges Kuppeln von Phenylalanindecarboxylase und Tyrosindecarboxylase an Phenylalanin und Tyrosin und sukzessives Kuppeln von Tyraminoxidase und anschließende Bestimmung des gebildeten Wasserstoffperoxids.

5. Verfahren zur selektiven Bestimmung nach Anspruch 2 zur Ermittlung von Phenylalanin und Tyrosin in einer Probe durch gleichzeitiges Kuppeln von Phenylalanin-4-monooxygenase in Gegenwart von 6-Methyl-5,6,7,8-tetrahydropteridin und Tyrosindecarboxylase an Phenylalanin und Tyrosin und sukzessives Kuppeln von Tyraminoxidase und anschließende Bestimmung des gebildeten Wasserstoffperoxids.

6. Verfahren zur selektiven Bestimmung nach Anspruch 2 zur Ermittlung von Phenylalanin und Tyrosin in einer Probe durch sukzessives Kuppeln von Tyrosindecarboxylase in einer Endkonzentration von 1 - 20

U/ml und Tyraminoxydase an Phenylalanin und Tyrosin und anschließende Bestimmung des gebildeten Wasserstoffperoxids.

7. Verfahren zur selektiven Bestimmung nach Anspruch 3 zur Ermittlung von Phenylalanin in einer Probe durch sukzessives Kuppeln von Phenylalanindecarboxylase und Tyraminoxidase an Phenylalanin und anschließende Bestimmung des gebildeten Wasserstoffperoxids.

8. Verfahren zur selektiven Bestimmung nach Anspruch 3 zur Ermittlung von Tyrosin in einer Probe durch sukzessives Kuppeln von Tyrosindecarboxylase und Tyraminoxidase an Tyrosin und anschließende Bestimmung des gebildeten Wasserstoffperoxids.

*Figure 1*

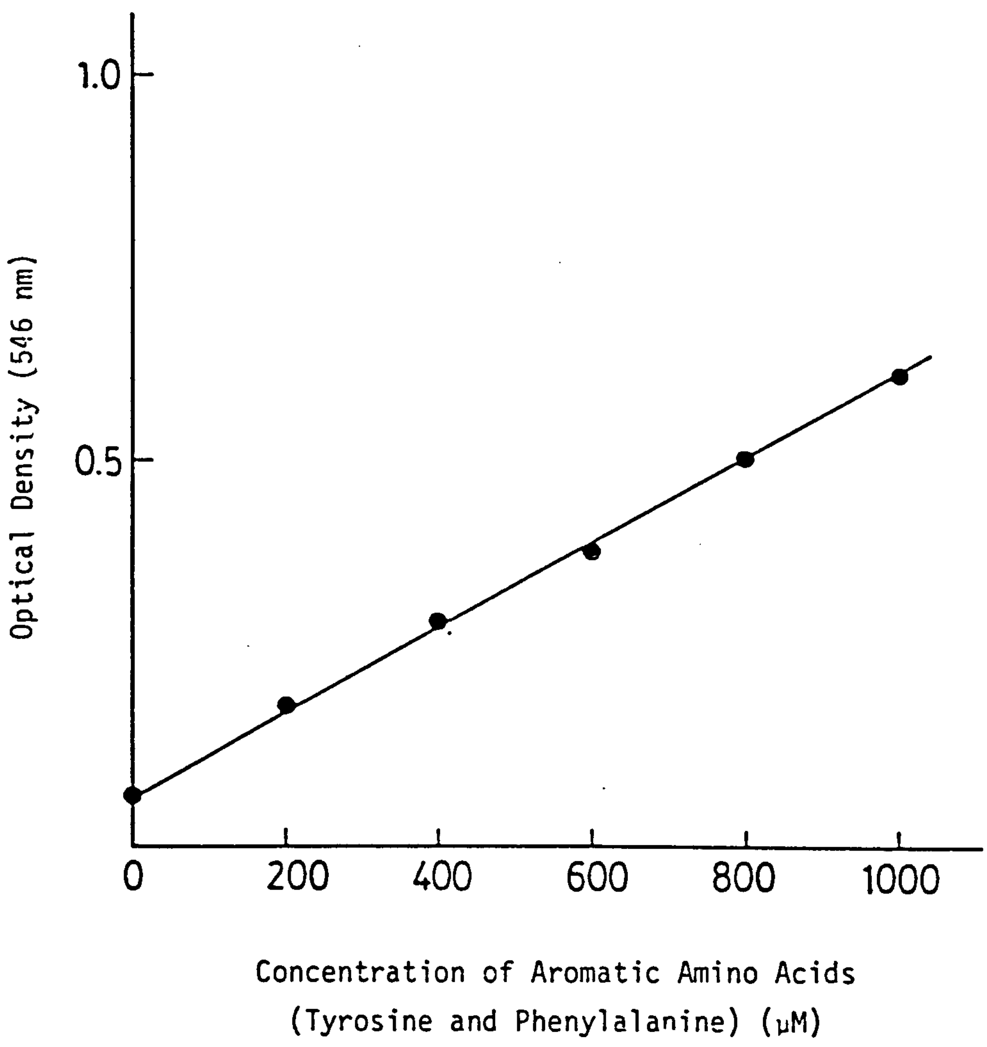
1.0
0.5
Optical Density (546 nm)
0
200
400
600
800
1000
Concentration of Aromatic Amino Acids
(Tyrosine and Phenylalanine) (μM)

*Figure 2*

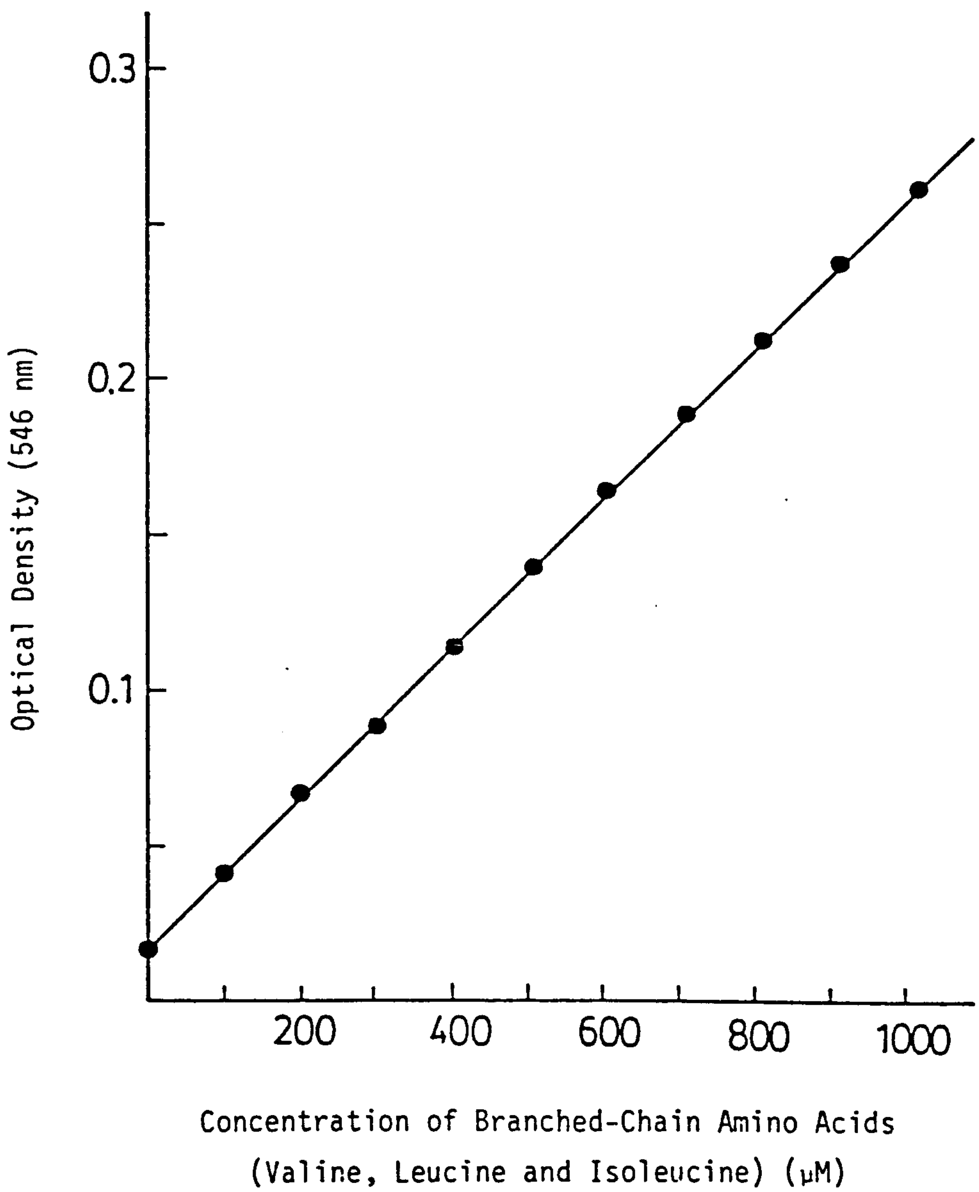
0.3
0.2
0.1
Optical Density (546 nm)
200
400
600
800
1000
Concentration of Branched-Chain Amino Acids
(Valine, Leucine and Isoleucine) (μM)

*Figure 3*

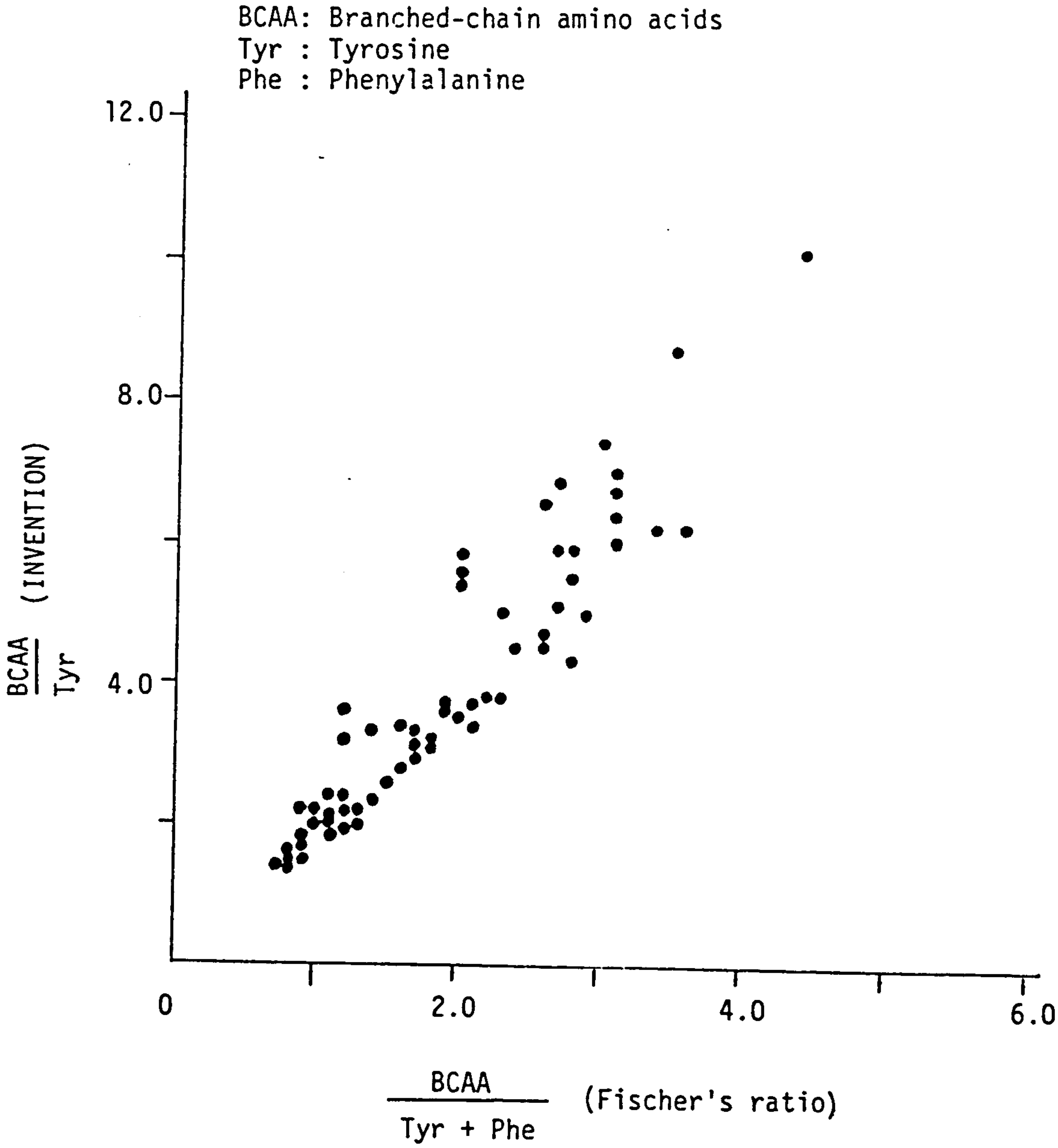
BCAA: Branched-chain amino acids
Tyr : Tyrosine
Phe : Phenylalanine
12.0
8.0
4.0
BCAA / Tyr (INVENTION)
0
2.0
4.0
6.0
BCAA / Tyr + Phe (Fischer's ratio)